# EUROPEAN PATENT SPECIFICATION

(11) **EP 3 612 837 B1**
(45) Date of publication and mention of the grant of the patent: **29.03.2023**
(21) Application number: 18724658.2
(22) Date of filing: 23.04.2018
(51) Int. Cl.: G01N 33/28

(54) **METHOD OF DETERMINING THE STABILITY RESERVE AND SOLUBILITY PARAMETERS OF A PROCESS STREAM CONTAINING ASPHALTENES BY JOINT USE OF TURBIDIMETRIC METHOD AND REFRACTIVE INDEX**
VERFAHREN ZUR BESTIMMUNG DER STABILITÄTSRESERVE UND LÖSLICHKEITSPARAMETER EINES PROZESSSTROMES MIT ASPHALTENEN DURCH GEMEINSAME VERWENDUNG EINES TURBIDIMETRISCHEN VERFAHRENS UND BRECHUNGSINDEX
PROCÉDÉ DE DÉTERMINATION LES PARAMÈTRES DE RÉSERVE DE STABILITÉ ET DE SOLUBILITÉ D'UN FLUX DE TRAITEMENT CONTENANT DES ASPHALTÈNES PAR UTILISATION CONJOINTE D'UN PROCÉDÉ DE TURBIDIMÉTRIE ET D'UN INDICE DE RÉFRACTION

(30) Priority: 21.04.2017 US 201715494199
(43) Date of publication of application: 26.02.2020
(73) Proprietor: Baker Hughes Holdings LLC, Houston, TX 77073 (US)
(72) Inventor: RESPINI, Marco, 26020 Casalmorano (IT); DELLA SALA, Giuseppe, Houston, TX 77073 (US); SANDU, Corina, Pearland, Texas 77584 (US); MEDINE, Gavin Mark, Houston, TX 77073 (NL); PINAPPU, Sai Reddy, Houston, Texas 77063 (US)
(74) Representative: Novagraaf Group
(86) International application number: PCT/US2018/028909
(87) International publication number: WO 2018/195543

(56) References cited:
- EP-A1- 3 147 649
- WO-A1-2013/070412
- US-A1- 2013 341 241
- US-A1- 2015 152 338
- US-A1- 2015 219 614
- J. S. Buckley ET AL: "Asphaltene precipitation and solvent properties of crude oils", Petroleum Science and Technology, Vol. 16, N? 3-4, 1998, pages 251-285, XP055030055, DOI: 10.1080/10916469808949783 Retrieved from the Internet: URL:http://baervan.nmt.edu/research_groups /petrophysics/aiche-97.pdf [retrieved on 2012-06-15]
- JILL S. BUCKLEY: "Predicting the Onset of Asphaltene Precipitation from Refractive Index Measurements", ENERGY & FUELS, vol. 13, no. 2, 1 March 1999 (1999-03-01), pages 328-332, XP055366837, WASHINGTON, DC, US. ISSN: 0887-0624, DOI: 10.1021/ef980201c

## Description

### BACKGROUND

### Field of the Invention

It is known in the art that solubility parameters of streams containing asphaltenes are related to the capability of the oil matrix to disperse/solvate asphaltenes and the tendency of asphaltenes to precipitate from the stream solvating them. Solubility parameters are a thermodynamic quantity related to cohesive energy and widely applied to determine solubilities of compounds in solvents. These solubility parameters are often respectively reported as a solubility blending number (related to the stream solubility parameter) and an insolubility number (solubility parameter of the asphaltenes in the stream).

The classic techniques reported in literature for the determination of the solubility parameters are inaccurate and their applicability is limited. For example, these traditional methods are limited and not as accurate to certain types of streams such as light crude oils with a low asphaltene content and heavy refinery residuals (thermally cracked or hydrocracked). They are also limited and not accurate for determining the impact of fluxants such as refinery distillates on the change of the matrix solubility parameters on these particular streams.

The refractive index can be utilized to measure the oil solubility parameter and calculate the solubility blending number starting from proprietary correlations based on experimental data. This method gives an approximate estimate of the stability reserve of asphaltenes in crude oils. However, the refractive index alone, without any other measurement, cannot give a direct and accurate estimation of the stability of thermally cracked streams such as FCC slurries, Eni slurry Technology unit (EST), HOil, LC finers and visbreakers under existing techniques.

J.S. Buckley et al. ("Asphaltene precipitation and solven properties of crude oil"; Petroleum Science and Technology, Volume 16, 1998; Issue 3-4) describes an improved prediction of the onset of asphaltene precipitation achieved by using refractive index (RI) to characterize crude oils and their mixtures with precipitants and solvents. Experimental measurements of RI for mixtures of several crude oils with the precipitant n-heptane, are reported at ambient conditions. Theoretical developments are described that will permit extension of these observations to reservoir conditions.

From US 2015/152338 A1 a method for reducing fouling from a quench oil is known. The method comprises obtaining at least one measurement related to light scattering properties for at least one quench oil with a probe, wherein the at least one quench oil comprises an amount of a precipitant.

Further, from US 2015/219614 A1 a method of determining a stability of a crude oil to control a process for refining the crude oil is known. The method comprises measuring a first refractive index (RI) value of the crude oil that does not comprise a solvent, wherein the first RI value is used to determine a first solubility parameter. Further, a second RI value of the crude oil at a point of asphaltene flocculation during a turbidimetric flocculation titration is measured, wherein the second RI value is used to determine a second solubility parameter.

WO 2013/070412 A1 refers to a method comprising precipitating an amount of asphaltenes from a liquid sample, determining one or more solubility characteristics, analyzing the characteristics, determining asphaltene content of the liquid sample, determining one or more asphaltene stability parameters, and correlating the asphaltene content and one of the asphaltene stability parameters to estimate sediment content of the liquid sample.

From EP 3 147 649 A1 a method of determining a settling rate of at least one foulant in oil-based fluids is known. The method comprises stirring an oil-based fluid during a turbidimetric flocculation titration of the oil-based fluid, stopping the solvent dosing at the onset of flocculation of the at least one foulant, stopping the stirring when at least two or more transmittance measurements are substantially similar, and measuring the transmittance of the oil-based fluid to determine a settling rate of the at least one foulant.

BUCKLEY JS, "Predicting the Onset of Asphaltene Precipitation from Refractive Index Measurements", Energy and Fuels, vol. 13, no. 2, March 1999, pages 328-332, ISSN: 0887-0624, DOI: 10.1021/ef980201c discloses an improved prediction of the onset of asphaltene precipitation achieved by using refractive index (RI) to characterize crude oils and their mixtures with precipitants and solvents.

Improvements in this field of technology are desired.

### SUMMARY

Disclosed is a method of determining solubility parameters and stability reserve of a process stream containing asphaltenes as set forth in claim 1. In certain illustrative embodiments, the solubility parameters are determined by measuring the refractive index of the process stream, RI and the refractive index at the flocculation onset of the asphaltenes (RIₒ), wherein the flocculation onset is determined via turbidimetric titration. The refractive index parameter at the flocculation onset, RIₒ is utilized as a direct measurement of the insolubility number, IN. The refractive index parameter of the stream containing asphaltenes, RI, is utilized as a direct measurement of the solubility blending number, SBₒ. The stability reserve is determined from the solubility blending number and the insolubility number (SBₒ/IN).

The measurements includes recovering precipitated asphaltenes at the flocculation onset, redispersing the precipitated asphaltenes in a solvent to form an asphaltenic solution, and measuring the refractive index parameters of the asphaltenic solution (RIₐ). The refractive index parameters is converted into a solubility blending number (SBₐ) of the precipitated asphaltenes.

The stability reserve is the ratio of the solubility blending number to the insolubility number. The precipitated asphaltenes can be recovered via one or more of filtration or centrifugation. The recovered precipitated asphaltenes can be washed with precipitant and then recovered again via one or more of filtration or centrifugation. The precipitated asphaltenes can be redispersed in the solvent at a ratio in the range from 1:1 solvent/asphaltenes to 10:1 solvent/asphaltenes. The process stream can include one or more of light crude oil, heavy crude oil and a refinery stream from the group comprising desalted crudes, vacuum tower bottoms, FCC slurries and heavy fuels. The process stream includes a live crude oil. The process stream can include a crude oil derivative from a refinery process. The process stream can include a light crude oil with an asphaltene content of 0.4% or greater. The process stream can include one or more residual thermal cracking streams from a refinery process. The residual thermal cracking streams can include FCC slurry, residual fuel oils, H-Oil, Eni Slurry Technology (EST) bottoms units and LC Finer bottoms and/or visbreaker residue. The process stream can include a residual hydrocracking stream from a refinery process. The process stream can include a refinery distillate stream containing no asphaltenes combined with a stream containing asphaltenes.

### BRIEF DESCRIPTION OF THE DRAWINGS

A better understanding of the presently disclosed subject matter can be obtained when the following detailed description is considered in conjunction with the following drawings, wherein:

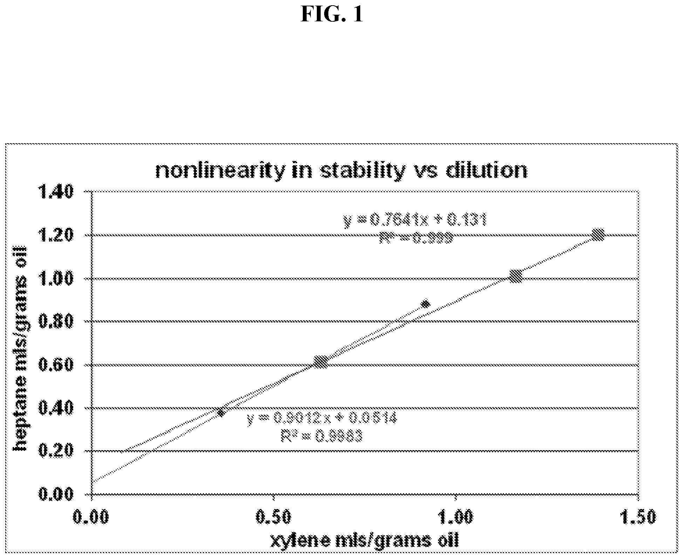

FIG. 1 is a graph showing nonlinearity of stability vs. dilution for a Heithaeus p-value three dilutions procedure in connection with the presently disclosed subject matter.

While the present invention will be described in connection with the preferred embodiment, it will be understood that it is not intended to limit the presently disclosed subject matter to that embodiment. The scope of the invention is defined by the appended claims.

### DETAILED DESCRIPTION

A method is provided for determining the solubility parameters for a variety of types of process streams, including crude oil and crude oil derivatives from refinery processing containing asphaltenes, and distillation products containing no asphaltenes when blended with asphaltene containing streams.

The present invention takes advantage of the joint use of turbidimetric detection of asphaltenes flocculation, which is used to determine and detect the onset flocculation of asphaltenes of the process stream coupled with the use of a refractive index to determine the process stream solubility parameters ( the solubility blending number and insolubility number).

The stability reserve of the asphaltenes is determined by the ratio of the solubility blending number ("SBₒ") (the capability to disperse the asphaltenes by the oil matrix surrounding the asphaltenes) to the insolubility number ("IN") (the asphaltenes insolubility). SBₒ and IN are determined from the refractive index ("RI") measurements.

The refractive index measurements is performed at the flocculation onset of the asphaltenes when a non-solvent (precipitant) is added. This RI measurement taken at the onset of asphaltene flocculation (RIₒ) allows one to determine the insolubility number (IN) of asphaltenes for the particular feedstock in a very clear and accurate way, offering improvement when compared to other methods. In some methods, such as optical methods, due to low amount of asphaltenes present in the feedstock subjected to the measurement, the onset flocculation determination may be difficult and inaccurate. By employing RI measurements at the flocculation onset of the asphaltenes, a direct way to determine the critical solubility parameter at which asphaltenes start to aggregate is possible and is a direct measurement of the IN.

The present invention provides improved reliability and accuracy in determining these solubility parameters and also extends the determination of the solubility parameters to process streams such as light crude oils with low asphaltene content, refinery heavy residuals from thermal cracking and hydrocracking and refinery distillates containing no asphaltenes.

As used herein, the term "process stream" broadly means streams from industrials processes such as tank blending optimization, refinery crude blending, refinery crude units, desalted crude, vacuum bottom towers resids, heavy fuels, coker, visbreakers, or a fluid originating from such streams. In addition, the same methodology or determinations can be performed during tank or terminal blending.

The process stream includes a live crude oil. The live crude oil can be produced, for example, from one or more offshore wells. The present invention can be applied to live crude oils on both the analytical side and for methods for refractive index measurements and the correlations between the oil solubility parameter and the refractive index. Certain commercially available refractometers are designed to work online on crude oils up to very high pressures. As such, the present invention has upstream applications, both for flow assurance when asphaltenes may flocculate due to phase change near the bubble point and for crude compatibility when streams from different blends are mixed. Upstream users can determine the solubility reserve of live crude oil samples and the flocculation point upon depressurization. Also, measurement of solubility parameters and calculating blending numbers can be useful in upstream situations where crude oil from different wells or different production zones within the same well are commingled. Such situations can occur, for example, in subsea applications where production from multiple wells are produced to a single flow line.

Since the RI technology has a higher degree of sensitivity due to its design, it can be leveraged to both very light asphaltenic containing streams as well as heavy and dark feedstocks. Applying refractive index determinations and coupling them with turbidimetric detection of asphaltene flocculation provides practical advantages and allow their use in controlling and detecting the stability reserve either on the bench or online for a variety or a larger pool of feedstocks than conventional methods. In addition, RI measurements are portable and testing is easy to perform. Thus, the present invention is suitable as a methodology that could be implemented in local/district labs.

In addition, the use of turbidimetric means, such as near infrared laser based determination of the asphaltenes flocculation onset, allows previously known methods (such as Heithaeus p-value) and similar ASTM methods like the D-7112 and D-7157 to be extended to a much wider range of streams having lower content of asphaltenes and with improved accuracy.

The solubility parameter of the process stream containing asphaltenes is determined by the use of the refractive index, whereby the critical solubility parameter IN (that is, solubility parameter at the asphaltenes flocculation onset) is determined by measurement of the refractive index at the flocculation point RIₒ as determined by turbidimetric titration. These are the most critical asphaltenes and are only a portion of the full content of asphaltenes; however, they are the most relevant for fouling and other asphaltene related issues.

In certain illustrative embodiments, when flocculation onset is reached (as identified by the turbidimetric titration) precipitant dosing is stopped and asphaltene aggregation is allowed to continue until completion (as identified by the flat minimum of the flocculation titration after the peak corresponding to precipitation onset). The critical asphaltenes can then be recovered either by filtration (using a filter from 0.2 to 10 microns, preferably 0.45 to 1 micron porosity) or by centrifugation (1000 to 20000 rpm, 1 minute to 1 hour).

The recovered asphaltenes can be then washed with hot precipitant and recovered again by filtration or centrifugation, to elimate trapped resins, waxes or other co-precipitated impurities. After the separation and washing, the asphaltenes are re-dispersed in a suitable aromatic solvent, typically xylene, in a ratio varying from 1:1 solvent/asphaltenes up to 10:1 solvent/asphaltenes, in certain illustrative embodiments. The refractive index of this asphaltenic solution RIₐ is measured and the solubility parameter of critical asphaltenes determined (SBₐ).

In certain illustrative embodiments, the refractive index can be directly measured during turbidimetric titration by using a fiber optic or miniature refractometer or any other refractometer that can be coupled with the turbidimeter. For example, near infrared may be utilized. The difference in the solubility parameter of the asphaltenes precipitated at the flocculation onset and the solubility parameter of the process stream with precipitant at the flocculation onset (SBₐ-IN) is indicative of the stabilizing/solvating role of the resins in the fluid on the critical asphaltenes. The solubility parameters are determined in both cases using the refractive index (RIₒ and RIₐ).

In certain illustrative embodiments, the solubility parameters of the full range of asphaltenes in the fluid (not restricted to the ones that are most critical and precipitate at the flocculation onset) can be determined by precipitating them from the fluid using an excess non-solvent (typically heptane), at ratios ranging from 10:1 to 40:1 heptane/fluid volume:weight. After recovering (via filtration or centrifugation) and washing with non-solvent in order to remove the trapped contaminants (such as resins and waxes), the solubility parameters of the asphaltenes can be measured by dispersing them in an aromatic solvent (SBₜₒₜₐₗ₋ₐ).

The solubility parameters of the solvating resins (SBₜₒₜₐₗ-ᵣ) can be determined in a similar way, for example by separating them from oil by known literature methods, dispersing them in an aromatic solvent after recovery and measuring the refractive index of the solution of the resins in the solvent.

By detecting the difference of SBₐ-SBₒ one can quanitfy and measure the efficiency and contribution provided by chemical additive programs designed to influence and improve both the SBₐ as well as SBₒ to have a positive impact on the overall stability reserve of the feed.

The impact of antifoulants (dispersants and asphaltenes inhibitors) on the solvation/stabilization by asphaltenes can also be tested by using the change in solubility parameters measured according to the presently disclosed subject matter. The impact on the overall stability reserve improvement can be refined by detecting and evaluating their influence on both SBₒ, SBₐ and/or SBᵣ.

In certain illustrative embodiments, SBₒ and IN are first determined in the field, for example, onsite at a refinery. This information may be sufficient to take action on additive investigation and recommendation. In the event that further and more specific testing is needed (which usually depends on the nature of the analyzed sample), additional laboratory determinations of SBₐ and SBᵣ can be performed. The field determinations of SB and IN can occur by both titration methods and RI determinations. This step can be followed by extension with additional parameters. The additional parameters can include, for example, recovering precipitated asphaltenes at the flocculation onset, redispersing the precipitated asphaltenes in a solvent to form an asphaltenic solution, measuring the refractive index parameters of the asphaltenic solution (RIₐ), and converting the refractive index parameters (RIₐ) into a solubility blending number (SBₐ) of the precipitated asphaltenes. In other illustrative embodiments, SBa and SBr can be routinely measured and introduced into the field procedure.

The present invention has a number of advantages when compared to prior art methods. For example, the solubility parameters can be determined more accurately. Traditional methods used multiple dilutions of the fluid containing asphaltenes in aromatic solvents at different oil to solvent ratios. This prior procedure can change the solvation layer of the asphaltenes and produce a serious bias in the results. Deviations from the linearity effects induced by dilution of different solvents were observed in the past. By introducing refractive index determinations on the pristine feedstocks with no dilution one allows a more representative determination of the solubility blending numbers as well as permits a user to quantify and assess the impact of chemical additives on the feedstock stability in a more reliable and accurate fashion.

The presently disclosed methods are also highly reliable due to the accuracy of turbidimetic titration in determining the true asphaltene flocculation onset coupled with the direct and accurate determination of the solubility parameters achievable by the use of the refractive index. For example, the reported error for p-value, SBn and IN from refinery experience and from round robin tests is 20% or greater, whereas the error of the presently disclosed methods is less than 2% in certain illustrative embodiments.

Furthermore, the presently disclosed methods are capable of measuring the solubility parameters for process streams with low asphaltene content, down to about 0.4 % asphaltene which is not achievable with current existing methods.

Furthermore, it is known in the art to determine SBn and In based on the Heithaeus p-value three dilutions procedure, whereby flocculation is measured on the pure stream containing asphaltenes (crude, residuum) and on the same sample diluted with an aromatic solvent (usually toluene). For example, this technique is generally described in U.S. Patent Publication No. 2013/0341241, published December 26, 2013, and assigned to Baker Hughes Incorporated.

However, the dilution makes the sample much more stable (more precipitant is needed, usually heptane) which makes the accurate determination of the flocculation onset very difficult or impossible, as the asphaltenes are extremely diluted at the flocculation onset many times.

This issue is avoided by the present invention as the SB_{feed} is measured directly on the process stream and the IN is measured as the RI at the flocculation onset. If the process stream is a solid at ambient temperature, it can be solvated with xylene and the RI measurement can be run on the diluted sample. As the presently disclosed method is able to accurately determine the flocculation onset of virtually any stream containing asphaltenes, the SBn and IN of a very wide range of streams can be measured.

The SBn and IN may be, in principle, determined by the three dilutions method, but this can fail sometimes or give wrong results. The basis of the experimental determination of the solubility parameters, SBn and IN, by the three dilutions method is based on the assumption that the dilution with aromatic solvent does not change the asphaltenes and their corresponding IN. If this is true, then the three dilutions plot is linear. This is a flawed assumption and there are typically different regression lines depending on the selected dilution and solvent range used to compensate for it. Consequently, the calculated SBn and IN depend upon the dilution range selected. A non-linear three dilutions plot is shown in Fig. 1 hereto.

The approximate estimate of the stability reserve of asphaltenes in crude oils provided by prior art techniques is based on the fact that SBn is determined with the RI and, on the assumption that on average, the asphaltenes insolubility number, IN, in crudes and residues before thermal cracking is 0.25. The SBn from RI measurements can be divided by 0.25 to get an estimate of the stability reserve. For thermally cracked streams, the IN value is drastically increased by the thermal cracking process in a way that depends on the severity of thermal cracking. Thus, the impact on IN is significant and needs to be better detected. In these cases unless there is a measurement of the IN value by independent techniques, an estimate on stability reserve cannot be given based solely on the RI.

The refractive index measurement alone, without any other measurement run on the asphaltenes containing feeds, can be utilized to measure the oil solubility parameter and calculate the solubility blending number starting from proprietary correlations based on experimental data. This gives an approximate estimate of the stability reserve of non thermally cracked asphaltene containing streams. If stability reserve is measured together with refractive index on the same stream, then the determination of the stability reserve becomes very accurate, and not simply an estimate, and can be extended to thermally cracked streams such as FCC slurries, H-Oil, LC Finer, Eni Slurry Technology EST unit and visbreakers.

While the disclosed invention has been described in detail in connection with a number of embodiments, it is not limited to such disclosed embodiments. The invention is defined by the attached claims.

## Claims

1. A method of determining solubility parameters and stability reserve of a process stream containing asphaltenes, the method comprising:
(a) determining the solubility parameters by measuring a refractive index parameter of the process stream (RI) and a refractive index parameter at the flocculation onset of the asphaltenes (RIₒ), wherein the flocculation onset is determined via turbidimetric titration, and wherein the measuring comprises:
(i) utilizing the refractive index parameter of the process stream (RI) as a direct measurement of a solubility blending number (SBₒ); and
(ii) utilizing the refractive index parameter at the flocculation onset of the asphaltenes (RIₒ) as a direct measurement of the insolubility number (IN);
(b) determining the stability reserve by calculating the stability reserve from the solubility blending number (SBₒ) and the insolubility number (IN), wherein the stability reserve is the ratio of the solubility blending number to the insolubility number; and
**characterised in that** the method further comprises:
(c) recovering precipitated asphaltenes at the flocculation onset, redispersing the precipitated asphaltenes in a solvent to form an asphaltenic solution, measuring the refractive index parameter of the asphaltenic solution (RIₐ), and converting the refractive index parameter (RIₐ) into a solubility blending number (SBₐ) of the precipitated asphaltenes;
wherein a difference in the solubility parameter of the asphaltenes precipitated at the flocculation onset and the solubility parameter of the process stream with precipitant at the flocculation onset (SBₐ - IN) is used to calculate the stabilizing/solvating effect of resins on the asphaltenes; and
wherein the process stream comprises a live crude oil.

2. The method of claim 1, wherein the precipitated asphaltenes are recovered via one or more of filtration or centrifugation.

3. The method of claim 2, wherein the recovered precipitated asphaltenes are washed with precipitant and then recovered again via one or more of filtration or centrifugation.

4. The method of claim 1, wherein the precipitated asphaltenes are redispersed in the solvent at a ratio in the range from 1:1 solvent/asphaltenes to 10: 1 solvent/asphaltenes.

## Patentansprüche

1. Verfahren zum Bestimmen von Löslichkeitsparametern und einer Stabilitätsreserve eines Prozessstroms, der Asphaltene enthält, das Verfahren umfassend:
(a) Bestimmen der Löslichkeitsparameter durch Messen eines Brechungsindexparameters des Prozessstroms (RI) und eines Brechungsindexparameters bei dem Ausflockungsbeginn der Asphaltene (RIₒ), wobei der Ausflockungsbeginn über Trübungstitration bestimmt wird, und wobei das Messen umfasst:
(i) Verwenden des Brechungsindexparameters des Prozessstroms (RI) als eine direkte Messung einer Löslichkeitsmischungszahl (SBₒ); und
(ii) Verwenden des Brechungsindexparameters bei Ausflockungsbeginn der Asphaltene (Riₒ) als eine direkte Messung der Unlöslichkeitszahl (IN);
(b) Bestimmen der Stabilitätsreserve durch Berechnen der Stabilitätsreserve aus der Löslichkeitsmischungszahl (SBₒ) und der Unlöslichkeitszahl (IN), wobei die Stabilitätsreserve das Verhältnis der Löslichkeitsmischungszahl zu der Unlöslichkeitszahl ist; und
**dadurch gekennzeichnet, dass** das Verfahren ferner umfasst:
(c) Rückgewinnen von ausgefällten Asphaltenen bei dem Ausflockungsbeginn, Redispergieren der ausgefällten Asphaltene in einem Lösungsmittel, um eine asphaltenische Lösung auszubilden, Messen des Brechungsindexparameters der asphaltenischen Lösung (RIₐ) und Umwandeln des Brechungsindexparameters (RIₐ) in eine Löslichkeitsmischungszahl (SBₐ) der ausgefällten Asphaltenen;
wobei ein Unterschied in dem Löslichkeitsparameter der Asphaltene, die bei dem Ausflockungsbeginn ausgefällt werden und dem Löslichkeitsparameter des Prozessstroms mit Fällungsmittel bei dem Ausflockungsbeginn (SBₐ - IN) verwendet wird, um die stabilisierende/solvatisierende Wirkung von Harzen auf die Asphaltene zu berechnen; und
wobei der Prozessstrom ein gashaltiges Rohöl umfasst.

2. Verfahren nach Anspruch 1, wobei die ausgefällten Asphaltene über eines oder mehrere von Filtration oder Zentrifugieren zurückgewonnen werden.

3. Verfahren nach Anspruch 2, wobei die zurückgewonnenen ausgefällten Asphaltene mit Fällungsmittel gewaschen werden und dann erneut über eines oder mehrere von Filtration oder Zentrifugieren zurückgewonnen werden.

4. Verfahren nach Anspruch 1, wobei die ausgefällten Asphaltene in dem Lösungsmittel in einem Verhältnis in dem Bereich von 1 : 1 Lösungsmittel/Asphaltene bis 10 : 1 Lösungsmittel/Asphaltene redispergiert werden.

## Revendications

1. Procédé de détermination de paramètres de solubilité et de réserve de stabilité d'un flux de processus contenant des asphaltènes, le procédé comprenant :
(a) la détermination des paramètres de solubilité en mesurant un paramètre indice de réfraction du flux de processus (RI) et un paramètre indice de réfraction au début de floculation des asphaltènes (RIₒ), dans lequel le début de floculation est déterminé par l'intermédiaire d'un titrage turbidimétrique, et dans lequel la mesure comprend :
(i) l'utilisation du paramètre indice de réfraction du flux de processus (RI) en tant que mesure directe d'un chiffre de solubilité de mélange (SBₒ) ; et
(ii) l'utilisation du paramètre indice de réfraction au début de floculation des asphaltènes (RIₒ) en tant que mesure directe du chiffre d'insolubilité (IN) ;
(b) la détermination de la réserve de stabilité en calculant la réserve de stabilité à partir du chiffre de solubilité de mélange (SBₒ) et du chiffre d'insolubilité (IN), dans lequel la réserve de stabilité est le rapport du chiffre de solubilité de mélange au chiffre d'insolubilité ; et
**caractérisé en ce que** le procédé comprend en outre :
(c) la récupération d'asphaltènes précipités au début de floculation, la redispersion des asphaltènes précipités dans un solvant pour former une solution asphalténique, la mesure du paramètre indice de réfraction de la solution asphalténique (RIₐ), et la conversion du paramètre indice de réfraction (RIₐ) en un chiffre de solubilité de mélange (SBₐ) des asphaltènes précipités ;
dans lequel une différence dans le paramètre de solubilité des asphaltènes précipités au début de floculation et du paramètre de solubilité du flux de processus avec précipitant au début de floculation (SBₐ- IN) est utilisée pour calculer l'effet de stabilisation/solvatation de résines sur les asphaltènes ; et
dans lequel le flux de processus comprend un pétrole brut.

2. Procédé selon la revendication 1, dans lequel les asphaltènes précipités sont récupérés par l'intermédiaire d'une ou plusieurs d'une filtration ou d'une centrifugation.

3. Procédé selon la revendication 2, dans lequel les asphaltènes précipités récupérés sont lavés avec du précipitant puis récupérés à nouveau par l'intermédiaire d'une ou plusieurs d'une filtration ou d'une centrifugation.

4. Procédé selon la revendication 1, dans lequel les asphaltènes précipités sont redispersés dans le solvant en un rapport dans la plage allant de solvant/asphaltènes de 1:1 à solvant/asphaltènes de 10:1.
